# EUROPÄISCHE PATENTSCHRIFT

(11) **EP 1 448 776 B1**
(45) Veröffentlichungstag und Bekanntmachung des Hinweises auf die Patenterteilung: **26.02.2014**
(21) Anmeldenummer: 02802651.6
(22) Anmeldetag: 06.11.2002
(51) Int. Cl.: C12N 15/10

(54) **VERFAHREN ZUR ISOLIERUNG VON NUKLEINSÄUREN**
METHOD FOR THE ISOLATION OF NUCLEIC ACIDS
PROCEDE D'ISOLEMENT D'ACIDES NUCLEIQUES

(30) Priorität: 06.11.2001 DE 10153957
(43) Veröffentlichungstag der Anmeldung: 25.08.2004
(73) Patentinhaber: QIAGEN GmbH, 40724 Hilden (DE)
(72) Erfinder: SINGER, Thorsten, 42697 Solingen (DE); WEBER, Martin, 42799 Leichlingen (DE)
(86) Internationale Anmeldenummer: PCT/EP2002/012384
(87) Internationale Veröffentlichungsnummer: WO 2003/040364

(56) Entgegenhaltungen:
- WO-A-95/01359
- DE-A- 19 903 507

## Beschreibung

Die vorliegende Erfindung betrifft ein vereinfachtes und schnelles Verfahren zur Isolierung von Nukleinsäuren insbesondere Plasmid DNA aus E. coli.

Verfahren zur Isolierung von Nukleinsäuren aus komplexen Ausgangsmaterialien sind an sich aus dem Stand der Technik bekannt und beinhalten im ersten Schritt die Lyse des biologischen Materials unter Verwendung eines Detergenz. Dabei wird dieser erste Schritt in Gegenwart von Enzymen durchgeführt, die für den Proteinabbau erforderlich sind.

In einem zweiten Schritt folgt im allgemeinen eine Extraktion der Nukleinsäuren mit geeigneten organischen Lösungsmitteln - wie z.B. Phenol und/oder Chloroform. Diesem Schritt folgt im allgemeinen eine Fällung der Nukleinsäuren mit Ethanol und die Dialyse der Nukleinsäuren. - So ist aus dem Stand der Technik u.a. ein Verfahren bekannt, in dem Plasmid-DNA verschiedenen Extraktions- und Detergens-Behandlungen unterzogen wird, um die Zellen zu lysieren, aus denen dann die Plasmid-Nukleinsäure erhalten wird und um Zellbestandteile und auch anderes Nukleinsäurematerial zu entfernen. Die reine Plasmid-DNA wird in einem abschließenden Schritt an Glaspulver in Abwesenheit einer chaotropen Substanz gebunden [M.A. Marko et al., Analytical Biochemistry 121, 382-387, 1982].

Die aus dem Stand der Technik bekannten Verfahren - wie z.B. die Isolierung von doppelsträngiger DNA - sind allerdings sehr arbeits- und zeitaufwendig. Die verhältnismäßig große Anzahl von Schritten, die erforderlich sind, um Nukleinsäuren aus derartigen Ausgangsmaterialien zu isolieren, vergrößern das Risiko der Kontamination bei der gleichzeitigen Bearbeitung verschiedener Proben. - Dieser Sachverhalt stellt insbesondere bei der Bearbeitung von klinischem Probenmaterial ein kaum kalkulierbares Risiko dar. Wenn die Nukleinsäure für einen anschließenden Nachweis auf Anwesenheit von Nukleinsäure, z.B. eines Krankheitserregers (z.B. ein Virus oder eines Bakteriums) mit dem Nukleinsäureamplifikationsverfahren, zum Beispiel mittels Polymerase-Kettenreaktion [PCR, Saiki et al., Science 230, 1985 1350] isoliert werden soll, dann ist dieses Risiko einer Kontamination mit fremder Nukleinsäure auf gar keinen Fall tolerierbar.

Des weiteren sind alternative Verfahren aus dem Stand der Technik bekannt, die jedoch ebenfalls mit dem Risiko der Probenkontamination behaftet sind.

So ist aus dem Stand der Technik ein Verfahren zur Isolierung von Gesamt-RNA aus Geweben und Zellkulturen bekannt [Analytical Biochemistry 162, 1987, 156]. Gemäß diesem Verfahren wird die RNA in einer einzigen Guanidiniumthiocyanat-Phenol-Chloroform-Mischung dem biologischen Ausgangsmaterial entzogen. Nach der Phasentrennung kann die RNA in einem brauchbaren Zustand innerhalb von 4 Stunden durch weiteres Verarbeiten der wässerigen Phase gewonnen werden.

Des weiteren ist aus dem Stand der Technik ein Verfahren zur Isolierung von DNA aus Geweben und Zelllinien beschrieben, bei dem die Zellen in einem Guanidinium-HCl enthaltendem Puffer dispergiert und in Ethanol gefällt werden [Analytical Biochemistry 162, 1987, 463]. Von diesem Verfahren ist bekannt, dass es für Kontaminationen anfällig ist, jedoch kann ein brauchbares NA-Produkt innerhalb weniger Stunden nach Bearbeitung der abgetrennten DNA isoliert werden.

Speziell im Hinblick auf Verfahren zur Isolierung von Plasmid-DNA mittels einer Plasmid-Matrix kann festgehalten werden, dass derartige Verfahren meist auf einer alkalischen Lyse basieren (Resuspension, alkalische Lyse, Neutralisation), wobei der Neutralisationspuffer chaotrope Salze zur Einstellung der Bedingungen zur Bindung der DNA an die Silica-Matrix enthält. Der Nachteil dieser aus dem Stand der Technik bekannten Verfahren wird durch einen voluminösen Niederschlag verkörpert, der zur Folge hat, dass das Lysat in einem weiteren sehr zeitaufwendigen Schritt (z.B. Zentrifugation oder Filtration) geklärt werden muss, bevor es mit der Matrix in Kontakt gebracht werden kann.

Das so geklärte Lysat wird dann in eine Säule überführt und dann unter Anlegen eines Vakuums oder auf dem Wege der Zentrifugation durch die Silica-Membran prozessiert.

Zur Abtrennung von Verunreinigungen wird die Membran mit einem alkoholhaltigen Puffer gewaschen und im Rahmen einer daran anschließenden Zentrifugations-Nakuumbehandlung von Alkoholresten befreit. Zuletzt wird die Nukleinsäure (DNA) mit einem sog. Niedrigsalzpuffer (z.B. 10 mM Tris-HCl, pH 8,5) eluiert.

Das grundlegende Prinzip dieser Verfahrensweise wird u.a. in der Europäischen Patentanmeldung Nr. 90 200 678.2 bzw. von R. Boom et. al. [R. Boom et al. J. Clin. Microbiol, 28 (3) (1990) 495] offenbart.

Die Offenlegungsschrift DE 199 03 507 beschreibt ebenfalls ein derartiges Verfahren zur Isolierung und Reinigung von Nukleinsäuren aus einer biologischen Probe, bei dem Kaliumacetat zur Neutralisierung und ein Silikagel ähnliches Trägermaterial verwendet werden, um die Nukleinsäuren Endotoxin frei oder an Endotoxin abgereichert zu isolieren.

Die vorliegende Erfindung stellt sich daher die Aufgabe, die Nachteile der aus dem Stand der Technik bekannten Verfahren zu überwinden und insbesondere ein Verfahren zur Verfügung zu stellen, das ohne die zeitaufwendige Klärung des Lysats auskommt.

Eine weitere Aufgabe der vorliegenden Erfindung besteht darin, ein Verfahren zur Verfügung zu stellen, das weitestgehend ohne Substanzen auskommt, die als "reizend" oder gar als gesundheitsschädlich angesehen werden.

Erfindungsgemäß werden diese Aufgaben dadurch gelöst, dass man nach der an sich bekannten alkalischen Lyse mit einem dafür aus dem Stand der Technik bekannten Puffer (z.B. 200 mM NaOH, 1% SDS) für den nachfolgenden Neutralisierungsschritt einen Neutralisierungspuffer auf der Basis eines Salzes einer Carbonsäure gemäß Anspruch 1 verwendet.

Überraschenderweise wurde festgestellt, dass sich durch die Anwendung des erfindungsgemäßen Verfahrens auch die Ausbeute gegenüber den entsprechenden - aus dem Stand der Technik bekannten - Verfahren erhöhen lässt

Als Carbonsäuren im Sinne der Erfindung werden zunächst gesättigte aliphatische Monocarbonsäuren, vorzugsweise C₁-C₆-Alkylcarbonsäuren verstanden, worunter Essigsäure, Propionsäure, n-Buttersäure, n-Valeriansäure, Isovaleriansäure, Ethyl-methyl-essigsäure (2-Methylbuttersäure), 2,2-Dimethylpropionsäure (Pivalinsäure), n-Hexansäure bevorzugt werden. Besonders bevorzugt werden Salze der Ameisensäure und/oder der Essigsäure eingesetzt.

Als ungesättigte Alkenyl-carbonsäuren im Sinne der Erfindung seien beispielsweise Acrylsäure (Propensäure), Methacrylsäure, Crotonsäure, iso-Crotonsäure sowie Vinylessigsäure genannt.

Daneben können gesättigte aliphatische C₂-C₆-Dicarbonsäuren, wie zum Beispiel Oxalsäure, Malonsäure, Bersteinsäure, Glutarsäure und/oder Adipinsäure eingesetzt werden.

Zur Lösung der erfindungsgemäßen Aufgaben eignen sich daneben aliphatische Hydroxi-di- und -tricarbonsäuren, worunter (2R, 3R)-(+)-Weinsäure, (2S, 3S)-(-)-Weinsäure, oder meso-Weinsäure bevorzugt werden.

Erfindungsgemäß finden Lithium-oder Natrium- oder Ammoniumsalze (NH₄⁺) Verwendung.

Daneben können Mischungen der beschriebenen Salze eingesetzt werden.

Durch den Einsatz von wässerigen Lösungen dieser Salze wird erfindungsgemäß eine Eintrübung des Lysats vermieden, womit sich der nachfolgende Klärungsschritt erübrigt.

Beispielhaft läuft das erfindungsgemäße Verfahren für die Isolierung von DNA - aus E. coli - wie folgt ab:

Das aus der Bakterienkultur gewonnene Pellet wird zunächst in einem geeigneten Puffer resuspendiert. Derartige Puffer sind aus dem Stand der Technik bekannt und auch kommerziell erhältlich. Ein geeigneter Puffer besteht aus einer wässerigen Lösung mit 50 mM Tris sowie 10 mM EDTA (pH 8,8). Der angegebene Puffer ist geeignet, um Bakterien zu resuspendieren - es ist jedoch auch der Einsatz jedweder anderen Salze, bzw. Puffersysteme, die im Bereich eines physiologischen pH-Wertes puffers, möglich.

Auch die für die anschließende Lyse einsetzbaren Puffer sind aus dem Stand der Technik bekannt und kommerziell erhältlich. Ein geeigneter Puffer besteht aus einer 200 mM Natriumhydroxid-Lösung enthaltend 1 % SDS - auch in diesem Fall ist der Einsatz anderer handelsüblicher Puffer, die ggf. andere Detergenzien enthalten - wie z.B. Triton - möglich.

Nach der Lyse über einen ausreichend langen Zeitraum erfolgt erfindungsgemäß die Zugabe einer wässerigen Lösung eines Acetatsalzes, wie z.B. einer 3 M wässerigen Ammoniumacetatlösung (pH 5,5). Hierbei ist auch der Einsatz von derivatisierten Acetaten - wie z.B. halogensubstituierter Acetate - möglich.

Nach der Zugabe des Alkohols, vorzugsweise eines verzweigen oder unverzweigten C₁ - C₃ - Alkohols, besonders bevorzugt *iso*-Propanol wird die so erhaltene Mischung mit einer Silica-Matrix in Kontakt gebracht, wobei die DNA quantitativ gebunden werden kann.

Zur Durchführung des erfindungsgemäßen Verfahrens eignen sich daneben auch andere alkoholische Hydroxygruppen aufweisende Verbindungen, wie z.B Polyethylenglykole. Bevorzugt werden dabei Polyethylenglykole mit einem Molekulargewicht in einem Bereich von 2000 bis 10000, besonders bevorzugt mit einem Molekulargewicht in einem Bereich von 4000 bis 8000 eingesetzt.

Die Abtrennung der Proteine erfolgt bei der Verwendung einer Silica-Membran als Matrix dadurch, dass der Anteil der in Lösung befindlichen Proteine nicht an die Silica-Matrix bindet, während ausgefällte Proteine irreversibel denaturiert sind und Proteine durch den Filtrationseffekt bei der Passage durch die Membran zurückgehalten werden.

Nach dem Durchleiten des Rohlysats durch die Silica-Matrix, wird - beispielsweise - die Membran mit einem Waschpuffer gewaschen.

Geeignete Waschpuffer sind ebenfalls aus dem Stand der Technik bekannt und kommerziell erhältlich. Die Anforderungen, die an geeignete Puffer zu stellen sind, bestehen lediglich darin, dass der Puffer gewährleisten muss, dass die Nukleinsäure nicht von der Matrix abgelöst wird. Allgemein genügt ein hoher Alkoholgehalt und optional leicht alkalischer pH, um die Autoproteolyse der DNA zu verhindern. Auch Puffer, die chaotrope Verbindungen enthalten - wie z.B. PB (QIAGEN GmbH, Hilden, Bundesrepublik Deutschland) - sind geeignet, solange sie die oben erwähnten Bedingungen erfüllen.

Nach dem Entfernen der Pufferrückstände - z.B. auf dem Wege der Zentrifugation erfolgt letztendlich die Elution der Nukleinsäure mittels eines geeigneten Elutionspuffers.

Auch diese Elutionspuffer sind aus dem Stand der Technik in hinreichender Weise bekannt sowie kommerziell erhältlich. Bevorzugt werden Puffer mit niedriger Salzkonzentration oder auch Wasser.

Erläuterung der Zeichnungen:

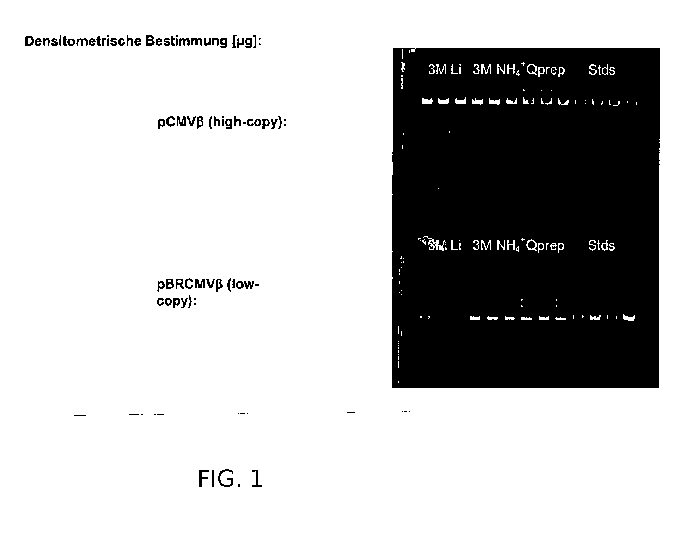

Fig. 1 zeigt das der densitometrischen Bestimmung zugrundeliegende Agarose-Gel aus Beispiel 2.

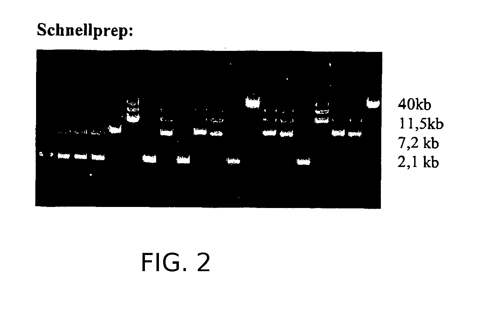

Fig. 2 zeigt das der densitometrischen Bestimmung zugrundeliegende Agarose-Gel aus Beispiel 3 - es wurden jeweils gleiche Volumina der jeweiligen Präparation 1 aufgetragen.

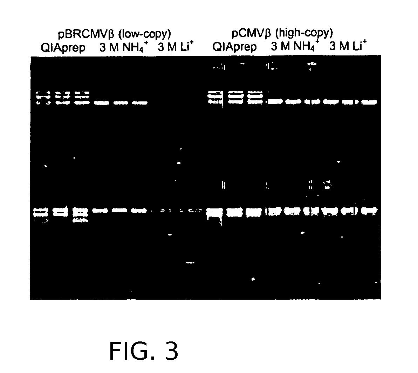

Fig. 3 zeigt das der densitometrischen Bestimmung zugrundeliegende Agarose-Gel aus Beispiel 4.

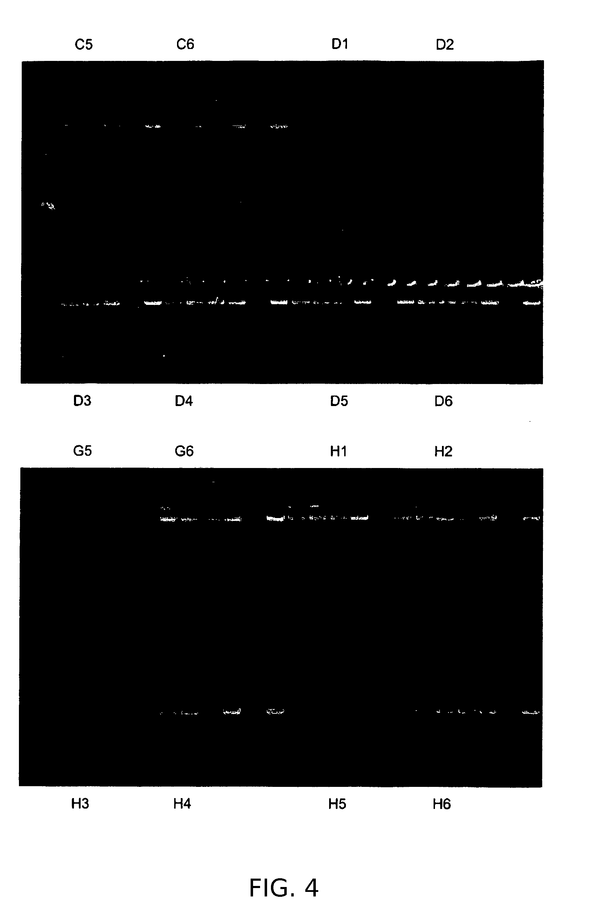

Fig. 4 zeigt die den densitometrischen Ausbeutebestimmungen zugrunde liegenden Agarose-Gele aus Beispiel 8.

Reihenfolge der Proben (v.l.n.r.): Erfindungsgemäßes Verfahren - 96-1 BR 3000
Erfindungsgemäßes Verfahren - 96-2 BR 3000
Stand der Technik (QIAprep Turbo 96) BR 3000
Erfindungsgemäßes Verfahren - 96-1 manuell leer
Erfindungsgemäßes Verfahren - 96-2 manuell

Die Randbezeichnungen geben die Position der aufgetragenen Proben im 96 Well Block entsprechend dem SBS-Standard an.

Die Gele zeigen für alle Präparationsmethoden gleichwertige Ergebnisse. Die Ausfälle der wells G5 und H5 liegen in der Heterogenität der aufgearbeiteten Genbank begründet und sind unabhängig von der angewendeten Präparationsmethode.

Die nachfolgenden Beispiele sollen die Erfindung verdeutlichen:

Vorbemerkungen:

Die angeführten Beispiele wurden nach dem folgenden Protokoll durchgeführt. Etwaige Abweichung sind gesondert angeführt.

Protokoll zur Isolierung von Plasmid-DNA aus E. coli im Minimaßstab: für 1,5 ml *E*. *coli* Kultur
(1) Bakterienpellet in 150 µl Respensionspuffer resuspendieren
(2) Zugabe von 150 µl Lysepuffer und vorsichtig mischen. Ca. 3 Minuten lysieren
(3) Zugabe von 150 µl Neutralisationspuffer und gut durchmischen (nicht vortexen!)
(4) Zugabe von 300 µl Isopropanol und gut durchmischen (nicht vortexen!)
(5) Rohlysat über eine Silicamembran-Säule geben und 1 min bei 14 000 Upm zentrifugieren.
(6) Waschen durch Zugabe von 750 µl 80 Vol.-proz. wässeriges Ethanol sowie 10 mM Tris und 1 min bei 14 000 Upm zentrifugieren
(7) Zum Entfernen von Pufferresten noch einmal 1 min bei 14 000 Upm zentrifugieren
(8) Elution mit 200 µl 10 mM Tris-Lösung (pH 8,5). Auf die Membran pipettieren, 1 min stehenlassen und durchzentrifugieren (1 min bei 14 000 Upm)

Durch das Wegfallen des Lysatklärungsschrittes verkürzt sich die Präparationszeit um 10 min auf die Hälfte der Zeit.

Bei den aufgeführten Beispielen wurden Referenz-Präparationen mit QIAprep als repräsentatives Beispiel für eine handelsübliche Silica-Methode mit einem chaotropen Bindepuffer durchgeführt. Die Referenz ist in den angeführten Beispielen durch "QIAprep" gekennzeichnet. (Bei der QIAprep-Methode wird aus einem zuvor geklärten Lysat die DNA in Gegenwart chaotoroper Salze, die in hoher Konzentration zugegen sind, an eine Silica-Membran gebunden und nach einem Reiningungsschritt von der Membran eluiert. Entsprechende Kits sind von der Fa. QIAGEN GmbH, Hilden, Bundesrepublik Deutschland, kommerziell erhältlich).

### Beispiel 1: Vergleich von 3 M NH₄OAc, pH 5,5 gegenüber 3 M LiOAc (Ac = COCH₃), pH 5,5 als Neutralisationspuffer; Einfluß des Kations.

Als Referenz wurde eine parallel eine Plasmid-Isolierung mit QIAprep durchgeführt.

| **DH5α/pCMVβ (High-Copy):** | | | | Verwendete Neutralisationspuffer (Schritt 3 im oben angegebenen Protokoll): | |
|---|---|---|---|---|---|
| **OD₂₆₀ [µg]:** | | | | | |
| | **3 M Li⁺** | **3 M NH₄⁺** | **QIAprep** | 3 M Li⁺ | 3 M Li-Acetat, pH 5,5 |
| | | | | 3 M NH₄⁺ | 3 M NH₄⁺-Acetat, pH 5,5 |
| 1 | 11,1 | 12,4 | 18,1 | | |
| 2 | 10,8 | 10,5 | 16,8 | | |
| 3 | 11,8 | 11,2 | 17,2 | | |
| **X** | **11,2** | **11,4** | **17,4** | | |
| | | | | | |

| **Densitometrische Bestimmung [µg]:** | | | | | |
|---|---|---|---|---|---|
| | | | | | |
| | **3 M Li⁺** | **3 M NH₄⁺** | **QIAprep** | | |
| 1 | 11,6 | 14,0 | 14,4 | | |
| 2 | 12,0 | 11,7 | 15,7 | | |
| 3 | 11,6 | 10,2 | 15,6 | | |
| **X** | **11,7** | **12,0** | **15,2** | | |

Die Ergebnisse zeigen, dass bei High-Copy-Plasmiden mit beiden Neutralisationspuffern eine gute Übereinstimmung zwischen den beiden Quantifizierungsmethoden erreicht wird. D.h.: es wird weitgehend nur Plasmid-DNA isoliert.

| **DH5α/pBRCMVβ (Low-Copy):** | | | |
|---|---|---|---|
| **OD₂₆₀ [µg]:** | | | |
| | **3 M Li⁺** | **3 M NH₄⁺** | **QIAprep** |
| 1 | 5,1 | 3,6 | 3,8 |
| 2 | 11,5 | 3,4 | 3,5 |
| 3 | 17,5 | 4,0 | 3,1 |
| **X** | **11,4** | **3,7** | **3,5** |

| | **3 M Li⁺** | **3 M NH₄⁺** | **QIAprep** |
|---|---|---|---|
| 1 | 0,7 | 1,8 | 2,7 |
| 2 | 0,9 | 2,0 | 3,0 |
| 3 | 0,4 | 2,1 | 2,7 |
| **X** | **0,7** | **2,0** | **2,8** |

Im Unterschied zu den High-Copy-Plasmiden zeigen die Low-Copy-Präparationen deutliche Unterschiede zwischen der Neutralisation mit Li- bzw. NH₄-Acetat. Eine gute Übereinstimmung beider Meßmethoden wird nur bei Neutralisation mit Ammoniumacetat, erreicht. Dieser Sachverhalt liefert einen Beleg dafür, dass das System damit wesentlich robuster wird und daher für den allgemeinen Einsatz besonders geeignet ist.

### Beispiel 2: Vergleich von 3 M NH₄OAc, pH 5,5 gegenüber anderen Ammoniumsalzlösungen als Neutralisationspuffer; Einfluß des Anions.

| **Ausbeuten [µg] nach OD₂₆₀:** | | | | | | | | |
|---|---|---|---|---|---|---|---|---|
| **Anion Konz.** | **Formiat** | | **HPO₄²⁻** | | **SO4²⁻** | | **Tartrat** | **H₂PO₄⁻** |
| | **1 M** | **3 M** | **1 M** | **3 M** | **1 M** | **3 M** | **1 M** | **1M** |
| 1 | 3,3 | 4,7 | 2,3 | 3,3 | 2,1 | 3,0 | 2,8 | 2,5 |
| 2 | 2,4 | 4,0 | 2,4 | 3,6 | 2,5 | 2,3 | 2,1 | 2,3 |
| **X** | **2,9** | **4,4** | **2,4** | **3,5** | **2,3** | **2,7** | **2,5** | **2,4** |

| | | | | | | | | |
|---|---|---|---|---|---|---|---|---|
| X = Mittelwert | | | | | | | | |

| **Ausbeuten [µg] nach Densitometrie:** | | | | | | | | |
|---|---|---|---|---|---|---|---|---|
| | | | | | | | | |

| **Anion Konz.** | **Formiat** | | **HPO₄²⁻** | | **SO4²⁻** | | **Tartrat** | **H₂PO₄⁻** |
|---|---|---|---|---|---|---|---|---|
| | **1 M** | **3 M** | **1 M** | **3 M** | **1 M** | **3 M** | **1 M** | **1M** |
| 1 | 1,3 | 4,8 | 1,8 | 1,0 | 1,8 | 0 | 2,8 | 0,6 |
| 2 | 0,8 | 4,5 | 1,3 | 1,4 | 2,1 | 0 | 1,7 | 0,3 |
| **X** | **1,1** | **4,7** | **1,6** | **1,2** | **2,0** | **0** | **2,3** | **0,5** |

| | | | | | | | | |
|---|---|---|---|---|---|---|---|---|
| X = Mittelwert | | | | | | | | |

Die Referenzausbeuten mit 3 M NH₄OAc, pH 5,5 wurden als 0% Fehlquantifizierung gesetzt und lagen im Mittel 5,2 µg (zwei separat angesetzte Pufferchargen, jeweils als Doppelbestimmung).

Die oben wiedergegebenen Ergebnisse belegen den Einfluss des Anions sowohl im Hinblick auf die Gesamtausbeuten, als auch bei der Reduzierung der photometrischen Überquantifizierung.

### Beispiel 3: Vergleich des erfindungsgemäßen Verfahrens (DirectPrep) für die Isolierung verschiedener Konstrukte aus verschiedenen Stämmen von E. coli.

Hierfür wurden vier typische Laborstämme ausgewählt und daraus Plasmide verschiedener Kopienzahl und unterschiedlicher Größen isoliert.

| **Erfindungsgemäßes Verfahren:** | | | | | | | |
|---|---|---|---|---|---|---|---|
| **Plasmid** | **Größe [kb]** | **Stamm** | **OD₆₀₀** | 1 | 2 | 3 | **X** |
| **Cosmid 9 (Low-Copy)** | **40** | **DH5α** | 2,8 | 10 | 8,3 | 7,4 | 8,6 |
| | | **HB101** | 3,2 | 8,1 | 9,1 | 9,2 | 8,8 |
| | | | | | | | |
| **pBRCMVβ (Low-Copy)** | **6,8** | **DH5α** | 3,4 | 7 | 4,8 | 4,9 | 5,6 |
| | | **HB101** | 3,4 | 5,9 | 4,1 | 5,6 | 5,2 |
| | | **TOP10F'** | 2,5 | 2,6 | 2,7 | 2,5 | 2,6 |
| | | **XL1blue** | 3,5 | 5,5 | 5,4 | 5,7 | 5,5 |
| | | | | | | | |
| **pCMVβ (High-Copy)** | **7,2** | **DH5α** | 2,3 | 12,1 | 10,2 | 11,8 | 11,4 |
| | | **HB101** | 2,6 | 8,2 | 9,9 | 8,3 | 8,8 |
| | | **TOP10F'** | 2,7 | 9,5 | 11,3 | 10,8 | 10,5 |
| | | **XL1blue** | 2,1 | 10,8 | 11,9 | 11,8 | 11,5 |
| | | | | | | | |
| **pTS64 (High-Copy)** | **11,5** | **DH5α** | 2,1 | 9,9 | 9,3 | 9,9 | 9,7 |
| | | **XL1blue** | 2,4 | 5,1 | 14,8 | 20,6 | 13,5 |
| | | | | | | | |
| **pUC21 (High-Copy)** | **3,2** | **DH5α** | 2,8 | 4,6 | 4,5 | 4,7 | 4,6 |
| | | **HB101** | 3,2 | 4,8 | 5,5 | 4,1 | 4,8 |
| | | **TOP10F'** | 2,1 | 3,4 | 3,8 | 3,7 | 3,6 |
| | | **XL1 blue** | 1,9 | 4,1 | 4 | 4,4 | 4,2 |

| **Verfahren aus dem Stand der Technik (QIAprep):** | | | | | | | |
|---|---|---|---|---|---|---|---|
| **Plasmid** | **Größe [kb]** | **Stamm** | **OD₆₀₀** | 1 | 2 | 3 | **X** |
| **Cosmid 9 (Low-Copy)** | **40** | **DH5α** | 2,8 | 10,9 | 10,8 | 9,8 | 10,5 |
| | | **HB101** | 3,2 | 11,3 | 5,2 | 11,5 | 9,3 |
| | | | | | | | |
| **pBRCMVβ (Low-Copy)** | **6,8** | **DH5α** | 3,4 | 4,1 | 4,2 | 4,7 | 4,3 |
| | | **HB101** | 3,4 | 6 | 9,2 | 8,6 | 7,9 |
| | | **TOP10F'** | 2,5 | 3,8 | 3,7 | 3,8 | 3,8 |
| | | **XL1blue** | 3,5 | 5,7 | 5,7 | 4,8 | 5,4 |
| | | | | | | | |
| **pCMVβ (High-Copy)** | **7,2** | **DH5α** | 2,3 | 14,7 | 13,6 | 14,2 | 14,2 |
| | | **HB101** | 2,6 | 10,3 | 10,6 | 9,9 | 10,3 |
| | | **TOP10F'** | 2,7 | 18,9 | 18,7 | 18,1 | 18,6 |
| | | **XL1blue** | 2,1 | 15,9 | 14,5 | 13,9 | 14,8 |
| | | | | | | | |
| **pTS64 (High-Copy)** | **11,5** | **DH5α** | 2,1 | 14,1 | 13,4 | 14,1 | 13,9 |
| | | **XL1blue** | 2,4 | 24,6 | 28,1 | 23,5 | 25,4 |
| | | | | | | | |
| **pUC21 (High-Copy)** | **3,2** | **DH5α** | 2,8 | 5,6 | 5,6 | 5,6 | 5,6 |
| | | **HB101** | 3,2 | 4,1 | 4,8 | 4,7 | 4,5 |
| | | **TOP10F'** | 2,1 | 5,1 | 5,2 | 5 | 5,1 |
| | | **XL1blue** | 1,9 | 6,4 | 5,8 | 5,7 | 6,0 |

Zur Bestimmung einer der Überquantifizierung wurden alle QIAprep-Werte auf 100% gesetzt, d.h. eine völlige Übereinstimmung zwischen OD₂₆₀ und densitometrischer Bestimmung vorausgesetzt. Damit wurden die folgenden relativen Werte für die Schnellpräparation erhalten:

| **Plasmid** | **Größe [kb]** | **Stamm** | **OD₆₀₀** | **Densitometrie** |
|---|---|---|---|---|
| **Cosmid 9 (Low-Copy)** | **40** | **DH5α** | 82 | 99 |
| | | **HB101** | 94 | 93 |
| | | | | |
| **pBRCMVβ (Low-Copy)** | **6,8** | **DH5α** | 128 | 86 |
| | | **HB101** | 66 | 120 |
| | | **TOP10F'** | 69 | 82 |
| | | **XL1blue** | 102 | 90 |
| **pCMVβ (High-Copy)** | **7,2** | **DH5α** | 80 | 119 |
| | | **HB101** | 86 | 93 |
| | | **TOP10F'** | 57 | 77 |
| | | **XL1blue** | 78 | 75 |
| | | | | |
| **pTS64 (High-Copy)** | **11,5** | **DH5α** | 70 | 100 |
| | | **XL1blue** | 53 | 64 |
| | | | | |
| **pUC21 (High-Copy)** | **3,2** | **DH5α** | 82 | 111 |
| | | **HB101** | 106 | 130 |
| | | **TOP10F'** | 71 | 101 |
| | | **XL1blue** | 70 | 94 |
| **Mittelwerte:** | | | **80** | **97** |

Im Mittel wurde eine Ausbeute von 80% der Ausbeuten der QIAprep-Präparationen nach OD₂₆₀ und nahezu 100% nach densitometrischer Auswertung erhalten.
Das Beispiel zeigt, dass das beschriebene neue Verfahren allgemein anwendbar ist und nahezu identische Ergebnisse im Vergleich zu bereits im Stand der Technik etablierten Methoden liefert.

Die Agarose-Gele zeigen außerdem eine weiteren Vorteil des erfindungsgemäßen Verfahrens: die DNA zeigte deutlich geringere Scherung der Plasmide, als bei den bisherigen Verfahren und liegt zu nahezu 100% als "supercoiled" Form vor.

### Beispiel 4: Stabilität und Qualität der isolierten Plasmid-DNA

Es wurde in einer Dreifachbestimmung je ein High- und Low-Copy-Plasmid isoliert und 20 h bei 37°C in Gegenwart eines DNase-Reaktionspuffers (Die Zusammensetzung derartiger Puffer ist aus dem Stand der Technik - wie z.B. aus molekularbiologischen Standardwerken - bekannt) inkubiert.
Dabei konnte keine Unterschied zwischen inkubierter und nicht-inkubierter Plasmid-DNA beobachtet werden, was die Abwesenheit von DNasen in der Präparation belegt. Dieses Beispiel zeigt deutlich, dass mit dem erfindungsgemäßen Verfahren isolierte DNA frei von kontaminierenden DNasen ist und die Lagerungsstabilität den etablierten Silica-Verfahren mit Lysatklärung und chaotroper Bindechemie gleichwertig. (Referenz für eine etablierte Präparationsmethode: QIAprep, Fa. QIAGEN). Im Unterschied zu dem etablierten Verfahren zeigt die nach dem neuen Verfahren isolierte DNA einen weit höheren Anteil an intakter "supercoiled" Form.

Testsequenzierungen ergaben identische Leseweiten für Plasmid-DNA, die nach dem neuen Verfahren und dem QIAprep-Verfahren isoliert wurde.

### Beispiel 5: Abhängigkeit von der eingesetzten Menge an Isopropanol zur Bindung

Es wurden je 1,5 ml DH5α/pBRCMVβ (Low-Copy) aufgearbeitet und ansteigende Mengen Isopropanol zur Bindung der Plasmid-DNA eingesetzt.

| **Ausbeuten [µg] nach OD₂₆₀:** | | | | | | | |
|---|---|---|---|---|---|---|---|
| | | | | | | | |
| **Vol % Isoprop.** | **28,6** | **33,3** | **37,5** | **41,2** | **44,4** | **47,4** | **50** |
| 1 | 2,2 | 2,6 | 2,8 | 2,9 | 4,1 | 5,5 | 14,1 |
| 2 | 3,8 | 2,2 | 2,7 | 3,1 | 6,0 | 9,3 | 18,2 |
| 3 | 2,5 | 2,6 | 2,4 | 3,5 | 5,4 | 7,0 | 13,7 |
| **Mittelwert** | **2,8** | **2,5** | **2,6** | **3,2** | **5,2** | **7,3** | **15,3** |

| **Ausbeuten [µg] nach Densitometrie:** | | | | | | | |
|---|---|---|---|---|---|---|---|
| | | | | | | | |
| **Vol % Isoprop.** | **28,6** | **33,3** | **37,5** | **41,2** | **44,4** | **47,4** | **50** |
| 1 | 1,1 | 2,6 | 2,8 | 2,9 | 2,8 | 2,1 | 4,3 |
| 2 | 2,7 | 1,8 | 3,2 | 2,9 | 3,9 | 3,8 | 4,0 |
| 3 | 2,4 | 2,9 | 2,4 | 2,6 | 3,1 | 3,3 | 3,6 |
| **Mittelwert** | **2,1** | **2,4** | **2,8** | **2,8** | **3,3** | **3,1** | **4,0** |

Beim Vergleich der man die erhaltenen Werte der photometrischen und densitometrischen Analyse, ist eine allmähliche Steigerung der Ausbeute bei zunehmendem Isopropanolanteil zu beobachten. Gleichzeitig fällt auf, dass diese Steigerung bei der photometrischen Messung sehr viel stärker ausfällt, als bei der Densitometrie. Da die densitometrische Bestimmung lediglich die enthaltene Plasmid-DNA erfaßt, die photometrische Analyse dagegen die Gesamtheit aller Nukleinsäuren, zeigt die zunehmende Diskrepanz zwischen OD-Bestimmung und Densitometrie bei höheren Isopropanolmengen einen höheren Grad an Verunreinigung. Die im Verfahren gewählten standardmäßig verwendeten 41,2 Vol% liefern einen Beleg für eine sehr gute Übereinstimmung beider Meßmethode bei größerer Gesamtausbeute im Vergleich zu den Ansätzen mit niedrigeren Alkoholmengen. Das zeigt, dass die so isolierte Plasmid-DNA einen geringeren Anteil an anderen kontaminierenden Nukleinsäuren enthält.

### Beispiel 6: Einfluss des verwendeten Alkohols

Es wurden je 1,5 ml DH5α/pCMVβ (Fa. Clontech) (High-Copy) aufgearbeitet und C₁-C₄-Alkohole auf ihre Eignung zur Bindung von Plasmid-DNA getestet. Alle Angaben in µg.

| **Ausbeute OD₂₆₀** | | | | | | | | |
|---|---|---|---|---|---|---|---|---|
| Alkohol | Isoprop | EtOH | MeOH | 1-ButOH | 1-ButOH | 2-ButOH | 2-ButOH | QIAprep |
| **Vol %** | **37,5** | **50** | **50** | **50** | **33,3** | **50** | **33,3** | **-** |
| 1 | 10,5 | 10,0 | 7,7 | 2,1 | 5,9 | 2,4 | 5,4 | 11,3 |
| 2 | 9,2 | 11,0 | 6,7 | 4,5 | 3,6 | 3,4 | 3,8 | 11,0 |
| **X** | **9,9** | **10,5** | **7,2** | **3,3** | **4,8** | **2,9** | **4,6** | **11,1** |

| **Ausbeute Densitometrie** | | | | | | | | |
|---|---|---|---|---|---|---|---|---|
| Alkohol | Isoprop | EtOH | MeOH | 1-ButOH | 1-ButOH | 2-ButOH | 2-ButOH | QIAprep |
| **Vol %** | **37,5** | **50** | **50** | **50** | **33,3** | **50** | **33,3** | **-** |
| 1 | 11,7 | 11,4 | 3,6 | 0,4 | 1,0 | 0,3 | 2,0 | 9,0 |
| 2 | 10,0 | 11,8 | 4,5 | 0,7 | 0,6 | 1,2 | 1,6 | 9,1 |
| **X** | **10,8** | **11,6** | **4,0** | **0,6** | **0,8** | **0,8** | **1,8** | **9,0** |

| **OD-Überquantifizierung [%]** | | | | | | | | |
|---|---|---|---|---|---|---|---|---|
| Alkohol | Isoprop | EtOH | MeOH | 1-ButOH | 1-ButOH | 2-ButOH | 2-ButOH | QIAprep |
| **Vol %** | **37,5** | **50** | **50** | **50** | **33,3** | **50** | **33,3** | **-** |
| 1 | 90 | 88 | 214 | 488 | 583 | 713 | 270 | 126 |
| 2 | 92 | 94 | 150 | 652 | 587 | 287 | 246 | 121 |
| **X** | 91 | 91 | 178 | 588 | 584 | 381 | 259 | 124 |

| | |
|---|---|
| Isoprop | Isopropanol |
| EtOH | Ethanol |
| MeOH | Methanol |
| 1-ButOH | Butan-1-ol |
| 2-ButOH | Butan-2-ol |

Die Ergebnisse zeigen deutlich, dass iso-Propanol und Ethanol, sowie auch Methanol zur selektiven Bindung von Plasmid-DNA an die Silica-Matrix für die Ausführung des erfindungsgemäßen Verfahrens geeignet sind.

### Beispiel 7: Verwendung von Polyethylenglykolen zur Einstellung der Bindebedingungen

-Es wurden je 1,5ml DH5α/pCMVβ (Fa. Clontech) (High-Copy) aufgearbeitet und Polyethylenglykole verschiedener Molekulargewichte auf ihre Eignung zur Bindung von Plasmid-DNA getestet. Es wurden, entsprechend dem eingangs beschriebenen Protokoll je 300 µl einer 40% (w/v) Lösung eingesetzt (alle Angaben in µg).

| **Ausbeute OD₂₆₀** | | | |
|---|---|---|---|
| | | | |

| **PEG** | **4000** | **6000** | **8000** |
|---|---|---|---|
| 1 | 4,6 | 3,5 | 5,7 |
| 2 | 4,6 | 5,1 | 5,3 |
| **X** | **4,6** | **4,3** | **5,5** |

| **Ausbeute Densitometer** | | | |
|---|---|---|---|
| | | | |

| **PEG** | **4000** | **6000** | **8000** |
|---|---|---|---|
| 1 | 4,4 | 2,3 | 2,8 |
| 2 | 4,0 | 4,1 | 5,0 |
| **X** | **4,2** | **3,2** | **3,9** |

| | | | |
|---|---|---|---|
| X = Mittelwert PEG = Polyethylenglykol 4000, 6000, 8000 = mittleres Molekulargewicht der Polyethylenglykole | | | |

Die experimentellen Befunde belegen, dass sich ebenfalls auch Polyethylenglykole verschiedener Molekulargewichte eignen sich sehr gut zur Einstellung der Bindebedingungen im Rahmen des erfindungsgemäßen Verfahrens. Die dabei erreichten Ausbeuten, sowie die Übereinstimmungen von photometrischer und densitometrischer Quantifizierung sind vergleichbar zu den z.B. mit iso-Propanol erreichten Ergebnissen.

### Beispiel 8: Anwendung des beschriebenen Verfahrens im Hochdurchsatzbereich

Es wurden je 1,25 ml DH10B/pUC19-Genbank (high-copy) in einem 96well Block angezogen und entsprechend dem unter "Vorbemerkungen" angegebenenen Protokoll aufgearbeitet. Zur Bindung der DNA wurde eine 96well Platte mit geeigneter Membrankombination verwendet. Das Verfahren wurde manuell, sowie auf einem BIOROBOT (Fa. QIAGEN) durchgeführt.
Als Referenz wurde mit dem QIAprep Turbo System (QIAGEN GmbH, Hilden, Bundesrepublik Deutschland) eine schon lange auf dem Markt befindliche Methode gewählt.

Alle Angaben in µg. Ausbeuten sind jeweils über alle 96 Proben gemittelt.

### Ausbeute Densitometer

| | | |
|---|---|---|
| **manuell** | **erfndungsgemäßes Verfahren 96** | **4,3** |
| | **Verfahren aus dem Stand der Technik (QIAprep Turbo 96)** | **6,0** |

### Ausbeute Densitometer

| | | |
|---|---|---|
| **BR3000** | **erfindungsgemäßes Verfahren 96** | **3,0** |
| | **Verfahren aus dem Stand der Technik (QIAprep Turbo 96)** | **2,8** |

BR3000 ...BIORobot 3000 (QIAGEN GmbH, Hilden, Bundesrepublik Deutschland)

Zur Überprüfung der Qualität der isolierten DNA wurden jeweils 24 der 96 Proben sequenziert und einer Phred20-Analyse unterzogen, die eine Aussage über die erhaltenen Leselängen und damit die Qualität erlaubt.

| | |
|---|---|
| **Stand der Technik (QIAprep Turbo):** | **524 Basenpaare** |
| **erfindungsgemäßes Verfahren:** | **505 Basenpaare** |

Unter Berücksichtigung der üblichen Schwankungen bei der Isolierung aus biologischen Systemen, zeigt sich das erfindungsgemäße Verfahren den aus dem Stand der Technik bekannten Verfahren in punkto Ausbeute und Qualität durchaus ebenbürtig. Das Wegfallen des Lysatklärungsschrittes erlaubt jedoch im Gegensatz zum Stand der Technik eine einfache gleichzeitige Aufarbeitung von 96 Klonen pro Platte. Daher ist das erfindungsgemäße Verfahren für den Hochdurchsatzbereich - manuell und vollautomatisiert- besonders geeignet.

Die vorliegende Erfindung betrifft ebenfalls eine Formulierung bzw. ein Kit zur Durchführung eines der anspruchsgemäßen Verfahren, insbesondere ein Kit enthaltend ein Lithium, Natrium oder Ammoniumsalz einer aliphatischen Carbonsäure - bevorzugt Ameisen- oder Essigsäure - ggf. in wässriger Lösung; einen aliphatischen Alkohol, insbesondere iso-Propanol und/oder Polyethylenglykol(e) mit einem Molekulargewicht in einem Bereich von 2.000 bis 10.000; ggf. einen Waschpuffer und einen Elutionspuffer.

## Patentansprüche

1. Verfahren zur Isolierung von Nukleinsäuren aus einer biologischen Probe, wobei die biologische Probe
i) einer alkalischen Lyse unterworfen wird,
ii) dem Lysat ein Neutralisationspuffer auf der Basis eines Salzes einer Carbonsäure zugegeben wird,
iii) dem neutralisierten Lysat Alkohol oder eine Verbindung, die eine alkoholische Hydroxygruppe aufweist, zugegeben wird,
iv) das Gemisch mit einer Silica-Matrix in Kontakt gebracht wird,
v) die Nucleinsäuren von der Silica-Matrix mittels eines Elutionspuffers eluiert werden,
**dadurch gekennzeichnet, dass** das Salz der Carbonsäure ausgewählt ist aus der Gruppe der Lithium-, Natrium- und Ammoniumsalze der Carbonsäuren.

2. Verfahren nach Anspruch 1, **dadurch gekennzeichnet, dass** man das Salz einer gesättigten aliphatischen Monocarbonsäure einsetzt.

3. Verfahren nach Anspruch 2, **dadurch gekennzeichnet, dass** man das Salz einer C₁-C₆-Alkylcarbonsäure einsetzt.

4. Verfahren nach Anspruch 3, **dadurch gekennzeichnet, dass** man das Salz der Essigsäure, Propionsäure, n-Buttersäure, n-Valeriansäure, Isovaleriansäure, Ethyl-methyl-essigsäure (2-Methylbuttersäure), 2,2-Dimethylpropionsäure (Pivalinsäure), und/oder n-Hexansäure einsetzt.

5. Verfahren nach Anspruch 1, **dadurch gekennzeichnet, dass** man als Salz einer Carbonsäure dasjenige einer ungesättigten Alkenyl-carbonsäure einsetzt.

6. Verfahren nach Anspruch 5, **dadurch gekennzeichnet, dass** man das Salz der Acrylsäure (Propensäure), Methacrylsäure, Crotonsäure, iso-Crotonsäure und/oder Vinylessigsäure einsetzt.

7. Verfahren nach Anspruch 1, **dadurch gekennzeichnet, dass** man als Salz einer Carbonsäure das Salz einer gesättigten aliphatischen C₂-C₆-Dicarbonsäure einsetzt.

8. Verfahren nach Anspruch 7, **dadurch gekennzeichnet, dass** man als Salz einer gesättigten aliphatischen C₂-C₆-Dicarbonsäure das Salz der Oxalsäure, Malonsäure, Bernsteinsäure, Glutarsäure und/oder Adipinsäure einsetzt.

9. Verfahren nach Anspruch 1, **dadurch gekennzeichnet, dass** man als Salz einer Carbonsäure das Salz einer aliphatischen Hydroxi-di- und -tricarbonsäure einsetzt.

10. Verfahren nach Anspruch 9, **dadurch gekennzeichnet, dass** man als Salz einer Carbonsäure das Salz einer aliphatischen Hydroxi-di-carbonsäure (2R, 3R)-(+)-Weinsäure, (2S, 3S)-(-)-Weinsäure und/oder meso-Weinsäure einsetzt.

11. Verfahren nach einem der Ansprüche 1 bis 10, **dadurch gekennzeichnet, dass** man als Salz der Carbonsäure ein Acetatsalz verwendet.

12. Verfahren nach Anspruch 11, **dadurch gekennzeichnet, dass** man Lithiumacetat oder Natriumacetat einsetzt.

13. Verfahren nach Anspruch 11, **dadurch gekennzeichnet, dass** man Ammoniumacetat einsetzt.

14. Verfahren nach einem der Ansprüche 1 bis 13, **dadurch gekennzeichnet, dass** man das Carbonsäuresalz ggf. zusammen mit anderen Hilfsstoffen in Form einer wässerigen Lösung einsetzt.

15. Verfahren nach einem der Ansprüche 1 bis 14, **dadurch gekennzeichnet, dass** die finale Konzentration des Carbonsäuresalzes im einem Intervall von 0.1 bis 5 M liegt.

16. Verfahren nach Anspruch 15, **dadurch gekennzeichnet, dass** die finale Konzentration des Carbonsäuresalzes in einem Intervall von 0.3 bis 2 M liegt.

17. Verfahren nach Anspruch 16, **dadurch gekennzeichnet, dass** das Carbonsäuresalz in der wässrigen Lösung final in 0.3 M Konzentration vorliegt.

18. Verfahren nach Anspruch 1, **dadurch gekennzeichnet, dass** das aus der Neutralisierung resultierende Reaktionsgemisch in Gegenwart eines verzweigten oder unverzweigten C₁-C₃-Alkohols mit der Silica-Matrix in Kontakt gebracht wird.

19. Verfahren nach Anspruch 18, **dadurch gekennzeichnet, dass** der Alkohol Ethanol ist.

20. Verfahren nach Anspruch 18, **dadurch gekennzeichnet, dass** der Alkohol *iso-*Propanol (Propan-2-ol) ist.

21. Verfahren nach Anspruch 1, **dadurch gekennzeichnet, dass** die Verbindung, die eine alkoholische Hydroxygruppe aufweist, Polyethylenglykol ist.

22. Verfahren nach Anspruch 21, **dadurch gekennzeichnet, dass** das mittlere Molekulargewicht der eingesetzten Polyethylenglykole in einem Intervall von 1.000 bis12.000 liegt.

23. Verfahren nach Anspruch 22, **dadurch gekennzeichnet, dass** das mittlere Molekulargewicht der eingesetzten Polyethylenglykole in einem Intervall von 2.000 bis10.000 liegt.

24. Verfahren nach Anspruch 23, **dadurch gekennzeichnet, dass** das mittlere Molekulargewicht der eingesetzten Polyethylenglykole in einem Intervall von 4.000 bis 8.000 liegt.

25. Verfahren nach einem der Ansprüche 1 bis 24, **dadurch gekennzeichnet, dass** der Elutionspuffer ein Puffer mit niedriger Salzkonzentration oder Wasser ist.

## Claims

1. Method of isolating nucleic acids from a biological sample, wherein the biological sample
i) is subjected to alkaline lysis,
ii) a neutralizing buffer based on a salt of a carboxylic acid is added to the lysate,
iii) alcohol or a compound having an alcoholic hydroxy group is added to the neutralized lysate,
iv) the mixture is contacted with a silica matrix,
v) the nucleic acids are eluted by means of an elution buffer from the silica matrix,
**characterized in that** the carboxylic acid salt is selected from the group of lithium, sodium and ammonium salts of carboxylic acids.

2. Method according to Claim 1, **characterized in that** the salt of a saturated aliphatic monocarboxylic acid is used.

3. Method according to Claim 2, **characterized in that** the salt of a C₁-C₆-alkylcarboxylic acid is used.

4. Method according to Claim 3, **characterized in that** the salt of acetic acid, propionic acid, n-butyric acid, n-valeric acid, isovaleric acid, ethylmethyl-acetic acid (2-methylbutyric acid), 2,2-dimethylpropionic acid (pivalic acid) and/or n-hexanoic acid is used.

5. Method according to Claim 1, **characterized in that** the salt of a carboxylic acid used is that of an unsaturated alkenyl-carboxylic acid.

6. Method according to Claim 5, **characterized in that** the salt of acrylic acid (propenoic acid), methacrylic acid, crotonic acid, iso-crotonic acid and/or vinylacetic acid is used.

7. Method according to Claim 1, **characterized in that** the salt of a carboxylic acid used is the salt of a saturated aliphatic C₂-C₆-dicarboxylic acid.

8. Method according to Claim 7, **characterized in that** the salt of a saturated aliphatic C₂-C₆-dicarboxylic acid used is the salt of oxalic acid, malonic acid, succinic acid, glutaric acid and/or adipic acid.

9. Method according to Claim 1, **characterized in that** the salt of a carboxylic acid used is the salt of an aliphatic hydroxy-di- and -tricarboxylic acid.

10. Method according to Claim 9, **characterized in that** the salt of a carboxylic acid used is the salt of an aliphatic hydroxy-di-carboxylic acid (2R,3R)-(+)-tartaric acid, (2S,3S)-(-)-tartaric acid, and/or meso-tartaric acid.

11. Method according to any of Claims 1 to 10, **characterized in that** the carboxylic acid salt used is an acetate salt.

12. Method according to Claim 11, **characterized in that** lithium acetate or sodium acetate is used.

13. Method according to Claim 11, **characterized in that** ammonium acetate is used.

14. Method according to any of Claims 1 to 13, **characterized in that** the carboxylic acid salt is optionally used together with other excipients in the form of an aqueous solution.

15. Method according to any of Claims 1 to 14, **characterized in that** the final concentration of the carboxylic acid salt is within a range from 0.1 to 5 M.

16. Method according to Claim 15, **characterized in that** the final concentration of the carboxylic acid salt is within a range from 0.3 to 2 M.

17. Method according to Claim 16, **characterized in that** the final concentration of the carboxylic acid salt in the aqueous solution is 0.3 M.

18. Method according to Claim 1, **characterized in that** the reaction mixture resulting from the neutralization is brought into contact with the silica matrix in the presence of a branched or unbranched C₁-C₃-alcohol.

19. Method according to Claim 18, **characterized in that** the alcohol is ethanol.

20. Method according to Claim 18, **characterized in that** the alcohol is *iso*-propanol (propan-2-ol).

21. Method according to Claim 1, **characterized in that** the compound having an alcoholic hydroxy group is polyethylene glycol.

22. Method according to Claim 21, **characterized in that** the average molecular weight of the polyethylene glycols used is within a range from 1,000 to 12,000.

23. Method according to Claim 22, **characterized in that** the average molecular weight of the polyethylene glycols used is within a range from 2,000 to 10,000.

24. Method according to Claim 23, **characterized in that** the average molecular weight of the polyethylene glycols used is within a range from 4,000 to 8,000.

25. Method according to any of Claims 1 to 24, **characterized in that** the elution buffer is a low salt concentration buffer or water.

## Revendications

1. Procédé pour l'isolement d'acides nucléiques d'un échantillon biologique, l'échantillon biologique
i) étant soumis à une lyse alcaline,
ii) le lysat étant additionné d'un tampon de neutralisation à base d'un sel d'un acide carboxylique,
iii) le lysat neutralisé étant additionné d'un alcool ou d'un composé qui présente un groupe hydroxy alcoolique,
iv) le mélange étant mis en contact avec une matrice de silice,
v) les acides nucléiques étant élués de la matrice de silice au moyen d'un tampon d'élution,
**caractérisé en ce que** le sel de l'acide carboxylique est choisi dans le groupe formé par les sels de lithium, de sodium et d'ammonium des acides carboxyliques.

2. Procédé selon la revendication 1, **caractérisé en ce qu'**on utilise le sel d'un acide monocarboxylique aliphatique saturé.

3. Procédé selon la revendication 2, **caractérisé en ce qu'**on utilise le sel d'un acide C₁-C₆-alkylcarboxylique.

4. Procédé selon la revendication 3, **caractérisé en ce qu'**on utilise le sel de l'acide acétique, propionique, n-butyrique, n-valérianique, isovalérianique, éthylméthylacétique (acide 2-méthylbutyrique), 2,2-diméthylpropionique (acide pivalique) et/ou n-hexanoïque.

5. Procédé selon la revendication 1, **caractérisé en ce qu'**on utilise comme sel d'un acide carboxylique celui d'un acide alcénylcarboxylique insaturé.

6. Procédé selon la revendication 5, **caractérisé en ce qu'**on utilise le sel de l'acide acrylique (propénoïque), méthacrylique, crotonique, iso-crotonique et/ou vinylacétique.

7. Procédé selon la revendication 1, **caractérisé en ce qu'**on utilise comme sel d'un acide carboxylique le sel d'un acide C₂-C₆-carboxylique aliphatique saturé.

8. Procédé selon la revendication 7, **caractérisé en ce qu'**on utilise comme sel d'un acide C₂-C₆-dicarboxylique aliphatique saturé le sel de l'acide oxalique, malonique, succinique, glutarique et/ou adipique.

9. Procédé selon la revendication 1, **caractérisé en ce qu'**on utilise comme sel d'un acide carboxylique le sel d'un acide hydroxydicarboxylique et hydroxytricarboxylique aliphatique.

10. Procédé selon la revendication 9, **caractérisé en ce qu'**on utilise comme sel d'un acide carboxylique le sel d'un acide hydroxydicarboxylique aliphatique acide (2R,3R)-(+)-tartrique, acide (2S,3S)-(-)-tartrique et/ou acide mésotartrique.

11. Procédé selon l'une quelconque des revendications 1 à 10, **caractérisé en ce qu'**on utilise comme sel de l'acide carboxylique un sel d'acétate.

12. Procédé selon la revendication 11, **caractérisé en ce qu'**on utilise l'acétate de lithium ou l'acétate de sodium.

13. Procédé selon la revendication 11, **caractérisé en ce qu'**on utilise l'acétate d'ammonium.

14. Procédé selon l'une quelconque des revendications 1 à 13, **caractérisé en ce qu'**on utilise le sel d'acide carboxylique le cas échéant ensemble avec d'autres adjuvants sous forme d'une solution aqueuse.

15. Procédé selon l'une quelconque des revendications 1 à 14, **caractérisé en ce que** la concentration finale du sel d'acide carboxylique se situe dans un intervalle de 0,1 à 5 M.

16. Procédé selon la revendication 15, **caractérisé en ce que** la concentration finale du sel d'acide carboxylique se situe dans un intervalle de 0,3 à 2 M.

17. Procédé selon la revendication 16, **caractérisé en ce que** le sel d'acide carboxylique se trouve en finalité dans la solution aqueuse en une concentration de 0,3 M.

18. Procédé selon la revendication 1, **caractérisé en ce que** le mélange réactionnel résultant de la neutralisation est mis en contact avec la matrice de silice en présence d'un alcool en C₁-C₃ ramifié ou non ramifié.

19. Procédé selon la revendication 18, **caractérisé en ce que** l'alcool est l'éthanol.

20. Procédé selon la revendication 18, **caractérisé en ce que** l'alcool est l'iso-propanol (propan-2-ol).

21. Procédé selon la revendication 1, **caractérisé en ce que** le composé qui présente un groupe hydroxy alcoolique est le polyéthylèneglycol.

22. Procédé selon la revendication 21, **caractérisé en ce que** le poids moléculaire moyen des polyéthylèneglycols utilisés se situe dans un intervalle de 1000 à 12 000.

23. Procédé selon la revendication 22, **caractérisé en ce que** le poids moléculaire moyen des polyéthylèneglycols utilisés se situe dans un intervalle de 2000 à 10 000.

24. Procédé selon la revendication 23, **caractérisé en ce que** le poids moléculaire moyen des polyéthylèneglycols utilisés se situe dans un intervalle de 4000 à 8000.

25. Procédé selon l'une quelconque des revendications 1 à 24, **caractérisé en ce que** le tampon d'élution est un tampon présentant une faible concentration en sel ou de l'eau.
